# EUROPÄISCHE PATENTSCHRIFT

(11) **EP 2 240 082 B1**
(45) Veröffentlichungstag und Bekanntmachung des Hinweises auf die Patenterteilung: **28.09.2011**
(21) Anmeldenummer: 09702031.7
(22) Anmeldetag: 19.01.2009
(51) Int. Cl.: A61B 10/02, A61B 10/00, A61M 1/36

(54) **VORRICHTUNG ZUR ENTNAHME VON BIOLOGISCHEM MATERIAL**
DEVICE FOR REMOVING BIOLOGICAL MATERIAL
DISPOSITIF DE PRÉLÈVEMENT DE MATIÈRE BIOLOGIQUE

(30) Priorität: 17.01.2008 DE 102008004977
(43) Veröffentlichungstag der Anmeldung: 20.10.2010
(73) Patentinhaber: Miltenyi Biotec GmbH, 51429 Bergisch Gladbach (DE)
(72) Erfinder: SCHREINER, Thomas, 50189 Eisdorf (DE)
(74) Vertreter: Hoppe, Georg Johannes
(86) Internationale Anmeldenummer: PCT/DE2009/000051
(87) Internationale Veröffentlichungsnummer: WO 2009/089829

(56) Entgegenhaltungen:
- EP-A- 1 642 533
- EP-A- 1 949 858
- WO-A-2007/021904
- WO-A-2008/121145
- US-A1- 2007 198 042

## Beschreibung

Die vorliegende Erfindung betrifft eine Vorrichtung und ein Verfahren zur Entnahme von biologischen Materialien, wie beispielsweise Knochenmark oder Blut aus einem Organismus. Gleichzeitig erlaubt die Vorrichtung eine sterile Bearbeitung (wie z. B. Zentrifugation) des biologischen Materials in der Vorrichtung.

Es gibt eine Vielzahl von Erkrankungen, bei denen biologische Materialien aus dem Körper eines lebenden oder toten Organismus entnommen werden. Dazu gehören beispielsweise Erkrankungen des hämatopoetischen und lymphatischen Systems wie akute und chronische Leukämien, Anämien, Lymphome, solide Tumoren wie Mamma-Karzinom, Schilddrüsen-Karzinom, malignes Melanom oder verschiedener innerer Organe wie Funktionsstörungen der Schilddrüse, Niereninsuffizienz, Leberinsuffizienz und andere. Diese Erkrankungen können die Entnahme von Blut, Urin, Lymphe und anderen Körperflüssigkeiten oder die Entnahme von Knochenmark erfordern.

Biologische Materialien bestehen meist aus verschiedenen Bestandteilen mit unterschiedlichen Eigenschaften. So ist Blut zusammengesetzt aus verschiedenen Zellarten, wie Leuko- und Erythrozyten und verschiedenen Stoffen wie Fetten und anderen Biomolekülen. Das Gleiche gilt für Knochenmark, das ebenfalls aus verschiedenen Zellarten, wie beispielsweise Knochenzellen (Osteoblasten und Osteoklasten), Stromazellen, blutbildende Stammzellen, Zellen des blutbildenden Systems in verschiedenen Reifestadien und Zellen des Immunsystems (B- und T- Lymphozyten und deren Vorläufer), sowie Fettzellen zusammengesetzt ist. Die Zellen des Knochenmarkes sind sowohl für die Bildung der Blutes und der lymphatischen Zellen, als auch für die Reparatur verschiedener Gewebe des Körpers und der Blutgefäße zuständig.

Das Knochenmark ist eine von Knochenbälkchen durchzogene weiche Masse (sogenannte Spongiosa), die sich in der Mitte der sie umgebenden harten Knochenbestandteile (Corticalis) befindet.

Bei verschiedenen Erkrankungen des blutbildenden Systems aber auch des Immunsystems wird eine Stammzelltransplantation durchgeführt. Am häufigsten handelt es sich bei derartigen Erkrankungen um solide Tumoren, Leukämien, Lymphome und Störungen der Blutbildung wie aplastische Anämie, für die Knochenmark verwendet wird. Zur Behandlung dieser Erkrankungen werden entweder autologe (körpereigene) Stammzellpräparate oder allogene (körperfremde) Stammzellpräparate verwendet. Der Erfolg einer Stammzelltransplantation hängt unter anderem davon ab, inwieweit bei allogenen Präparaten Spender/-in und Empfänger/-in genetisch kompatibel sind, d. h. in den Genen des Human Leukocyte Antigen (HLA) Komplexes übereinstimmen. Weisen Spender- und Empfängermaterial zu große Unterschiede auf, können schwere Nebenwirkungen wie Abstoßung des Transplantates oder die "Graft versus Host Disease" (GvHD) auftreten, die zu schweren Problemen und schließlich zum Tod des Patienten führen können.

Der Erfolg einer Transplantation hängt aber auch von der Qualität des zu übertragenden biologischen Materials, wie beispielsweise dem Blut oder des Knochenmarks ab. Während eine Blutentnahme relativ unproblematisch ist, gelten für eine Entnahme von Knochenmark höhere Anforderungen. Da die Entnahme mit Schmerzen verbunden ist, findet die Entnahme von Knochenmark unter Narkose oder örtlicher Betäubung statt. Zudem muss die Corticalis durchdrungen werden, um schließlich an das Knochenmark zu gelangen. Dies erfordert besondere Geräte, die dazu geeignet sind, den Knochen aufzubohren und Platz für eine Kanüle zu schaffen, die groß genug ist, um die Zellen des Knochenmarks bei der Entnahme nicht zu beschädigen. Diese Entnahme erfolgt entweder rein manuell durch Ansaugen des Knochenmarkes mittels einer Spritze oder mit Hilfe von Saugpumpen. Derartige Geräte sind dem Fachmann bekannt.

Aus der US 2007/0198042 A1 sind eine Vorrichtung zur Entfernung und/oder Injektion von Knochenmark und ein diese Vorrichtung umfassendes System bekannt.

Derzeit wird das Knochenmark häufig unter Verwendung einer sogenannten Jamshidi Kanüle mit einer Spritze entnommen. Dabei handelt es sich um eine Hohlnadel mit abgerundeter Spitze, einen Trocar (auch Mandrin genannt) mit scharf geschliffener Spitze und einen Haltegriff mit einem Adapter für die Spritze (Luer Adapter). Dieses mehrteilige, zusammengesetzte Werkzeug wird von Hand mit der scharfen Spitze durch die harte Knochenwand gebohrt. Danach wir der Trocar entnommen, wodurch die Hohlnadel alleine im Knochenmark verbleibt. Dann wird eine Spritze auf den Adapter aufgesetzt und ein relativ kleines Volumen Knochenmark wird in die Spritze aufgezogen.

Damit das Knochenmark nicht gerinnt, muss in der Spritze vor der Entnahme eine gerinnungshemmende Substanz aufgezogen werden. Dies geschieht üblicherweise nicht in einem den Grundlagen der Arzneimittelherstellung entsprechenden Raum, sondern bestenfalls in der Umgebung eines Operationssaales, was das Risiko einer Verunreinigung des Knochenmarks mit sich bringt.

Da das Volumen, das mit einer Spritze aufgenommen werden kann, in den meisten Fällen nicht für eine Transplantation ausreicht, muss die Spritze an verschiedenen Punkten des Knochens angesetzt werden, um genügend Knochenmark zu erhalten. Dieses mehrmalige erneute Ansetzen der Spritze und nachfolgendes Entleeren des Knochenmarks aus der Spritze in ein Gefäß birgt die Gefahr der Kontamination durch in der Luft herumfliegende Partikel und Mikroorganismen. Letztere sind für die transplantierten Patienten besonders problematisch, da ihr Immunsystem vor der Transplantation stark geschwächt wurde, damit das neu übertragene Knochenmark seine Funktion aufnehmen kann. Da die Patienten nun mindestens zehn Tage lang ganz ohne Leukozyten bzw. vier bis 50 Wochen lang mit einer stark verminderten Immunabwehr leben müssen, kann jede Art von Kontamination zu einem lebensbedrohlichen Risiko für sie werden. Die Kontamination kann vor Verabreichung des Knochenmarks nicht erkannt werden, da diese innerhalb weniger Stunden nach der Entnahme des Knochenmarks durchgeführt wird, also zu einer Zeit, in der noch keine Ergebnisse über eine eventuell vorhandene Kontamination des zu transplantierenden Knochenmarks vorliegen können.

Desgleichen ist es für den das Knochenmark entnehmenden Operateur sehr umständlich, immer wieder neue Spritzen mit gerinnungshemmenden Substanzen zu füllen und mit der Kanüle im Knochenmark zu verbinden und den Vorgang der Knochenmarksentnahme fortzusetzen.

### Beschreibung der Erfindung

Die genannten Probleme werden mit der erfindungsgemäßen Vorrichtung zur Entnahme und Prozessierung eines fließfähigen biologischen Materials aus einem Organismus gelöst.

Die erfindungsgemäße Vorrichtung zur Entnahme biologischen Materials aus einem Organismus ist in einer bevorzugten Ausführungsform auch zur Auftrennung der biologischen Probe in mindestens zwei seiner Bestandteile geeignet.

Die Vorrichtung weist ein Entnahmemittel zur Entnahme biologischen Materials aus einem Organismus auf. Die Entnahme erfolgt durch Erzeugung eines negativen Drucks (Soges, Unterdrucks) gegenüber dem im Organismus herrschenden Druckes. Das Entnahmemittel erlaubt auch die Abgabe des Materials in ein Aufnahmemittel der erfindungsgemäßen Vorrichtung zur Aufnahme des Materials aus dem Organismus und zur späteren Prozessierung der biologischen Probe (etwa durch Zentrifugation etc.). Die Abgabe erfolgt ebenso wie die Aufnahme durch Erzeugung einer Druckdifferenz, nämlich hier durch Erzeugung eines positiven Drucks gegenüber der Umgebung.

In einer Ausführungsform, in der die Vorrichtung auch zur Auftrennung der biologischen Probe in mindestens zwei seiner Bestandteile geeignet ist, ist das Aufnahmemittel bevorzugt derart ausgestaltet, dass das biologische Material im Aufnahmemittel in mindestens zwei seiner Bestandteile trennbar ist, etwa durch Zentrifugation. Beispielsweise kann Blut somit im Aufnahmemittel durch Zentrifugation des Aufnahmemittels in Plasma und Erythrozyten aufgetrennt werden. Für die Herstellung des Aufnahmemittels geeignete Materialien sind dem Fachmann bekannt.

Dem Entnahmemittel der Vorrichtung kann in einer Ausführungsform ein Umleitungsmittel zugeordnet sein, dass am Entnahmemittel beispielsweise mittels einer Schlauchverbindung angeordnet ist oder einstückig mit dem Entnahmemittel ausgeformt ist. Die einstückige Ausformung birgt den Vorteil eines verminderten Kontaminationsrisikos. Dieses Umleitungsmittel dient der wahlweisen lumigen Verbindung des Entnahmemittels mit dem genannten Aufnahmemittel, mit einem Penetrationsmittel zur Penetration in den Organismus, und mit einem Vorratsmittel zur Bevorratung einer Substanz, insbesondere zur Bevorratung eines Antikoagulanz, verbindbar ist. Diese wahlweise lumige Verbindung wird durch bestimmte Funktionsstellungen des Umleitungsmittels ermöglicht.

Das Umleitungsmittel ist derart ausgestaltet, dass das Entnahmemittel in jeweils einer möglichen Funktionsstellung wahlweise entweder nur mit dem Vorratsmittel und dem Aufnahmemittel, oder nur mit dem Penetrationsmittel lumig verbunden ist. Alternativ kann das Umleitungsmittel in eine Position gebracht werden, in der das Entnahmemittel von den anderen Bestandteilen der Vorrichtung abgekoppelt ist. In dieser Position des Umleitungsmittels sind nur das Vorratsmittel, das Aufnahmemittel und das Penetrationsmittel lumig miteinander verbunden (keine Funktionsstellung). In dieser Stellung des Umleitungsmittels kann das Entnahmemittel beispielsweise entfernt und ausgetauscht werden.

Die erfindungsgemäße Vorrichtung bildet in einer Ausführungsform ein einseitig offenes System, das nur über das Penetrationsmittel (und ggf. am Umleitungsmittel) nach außen geöffnet ist. Damit wird ein steriles System bereitgestellt und die Gefahr des Eindringens von Verunreinigungen vermindert. Vorteilhafter Weise ist die Vorrichtung aus einem für Mikroorganismen unpermeablen Material gefertigt. In einer weiteren Ausführungsform ist an der Vorrichtung mindestens ein Anschlussmittel (z. B. in Form eines Luer Konnektors) zum Anschließen mindestens einer weiteren Komponente vorgesehen.

Die erfindungsgemäße Vorrichtung kann zur Entnahme und Prozessierung eines beliebigen fließfähigen biologischen Materials aus einem Organismus verwendet werden. Insbesondere kann die Vorrichtung für jedwede Körperflüssigkeit, wie Blut, Urin, Lymphe oder Liquor, aber auch für Gewebe, Zellen oder Fraktionen oder Bestandteile hiervon verwendet werden. Besonders geeignet ist die Vorrichtung für die Entnahme von Knochenmark. In einer bevorzugten Ausführungsform ist die erfindungsgemäße Vorrichtung somit eine Knochenmarkentnahme- und -aufarbeitungsvorrichtung, in einer anderen bevorzugten Ausführungsform ist die erfindungsgemäße Vorrichtung somit eine Blutentnahme- und -aufarbeitungsvorrichtung. Organismen, aus denen mit der Vorrichtung biologisches Material entnommen werden kann, sind insbesondere Säugetiere, bevorzugt der Mensch.

Bevorzugter Weise ist das Umleitungsmittel zwischen dem Entnahmemittel und dem Penetrationsmittel angeordnet. Ebenso ist bevorzugter Weise das Umleitungsmittel zwischen dem Vorratsmittel und dem Aufnahmemittel angeordnet. In einer bevorzugten Ausführungsform ist somit das mit dem Entnahmemittel verbundene Umleitungsmittel über Leitungsmittel lumig auf einer ersten Seite mit dem Penetrationsmittel, auf einer zweiten Seite mit dem Vorratsmittel und auf einer dritten Seite mit dem Aufnahmemittel verbunden.

Um die Fließrichtung des biologischen Materials festzulegen, weist die Vorrichtung in einer weiteren bevorzugten Ausführungsform im Leitungsmittel zwischen dem Vorratsmittel und dem Umleitungsmittel ein erstes Ventil, z. B. ein erstes Rückschlagventil auf, dass so angeordnet ist, dass die bevorratete Substanz nur vom Vorratsmittel in Richtung Umleitungsmittel, nicht jedoch in umgekehrter Richtung fließen kann.

Ebenso kann in einer weiteren bevorzugten Ausführungsform, die ggf. auch das erste Ventil aufweist, zwischen dem Umleitungsmittel und dem Aufnahmemittel ein zweites Ventil Ventil, z.B. ein zweites Rückschlagventil im Leitungsmittel angeordnet sein, so dass das entnommene biologische Material und/oder die Vorratsmittel bevorratete Substanz nur vom Umleitungsmittel in Richtung Aufnahmemittel fließen kann.

Das Vorhandensein derartiger Ventile ist insbesondere dann vorteilhaft, wenn die Vorrichtung zur Entnahme und ggf. Verarbeitung (Auftrennung) von Knochenmark dienen oder dazu verwendet werden soll, für diesen Zweck hohe Drücke erzeugt werden müssen, die das Risiko bergen, dass eine in der Vorrichtung befindliche Substanz, wie biologisches Material oder im Vorratsmittel befindliches Antikoagulanz, im Lumen in eine nicht gewünschte Richtung bewegt wird.

Um ausreichende Sterilität zu gewährleisten ist bevorzugter Weise in dem Leitungsmittel mindestens ein Filter zum Filtern von Mikroorganismen und/oder stückigem Material angeordnet.

Insbesondere die hier verwirklichte Kombination von Entnahme-, Umleitungs-, Penetrations-, Vorrats- und Aufnahmemittel mit mindestens einem Ventil und mindestens einem Filter in eine Vorrichtung ermöglicht eine kontaminationsfreie Entnahme und auch Prozessierung von biologischem Material, so dass die Vorrichtung auch beispielsweise in Operationssälen verwendet werden kann. Auf die Verwendung zertifizierter Reinräume zur Verarbeitung (z. B. Auftrennung) des biologischen Materials kann somit verzichtet werden. Abgetrennte Bestandteile des biologischen Materials können - ggf. nach Behandlung, wie etwa nach dem Zusatz mindestens eines Arzneimittels - dem Organismus, aus dem es ursprünglich entnommen worden ist, oder einem anderen Organismus, zugefügt werden.

In einer weiteren Ausführungsform der Vorrichtung ist das Entnahmemittel als Spritze oder Pumpe ausgeformt, mit der der für den Transport des biologischen Materials und/oder der bevorrateten Substanz im Leitungsmittel nötige Druckdifferenz erzeugt werden kann. Das

Umleitungsmittel ist bevorzugt als Dreiwegehahn (Dreiwegeventil, 3-way stop cock) ausgeformt. Um die Handhabbarkeit der Vorrichtung zu erleichtern, ist das Leitungsmittel bevorzugt als flexibler Schlauch ausgeformt. Als Penetrationsmittel wird bevorzugt eine Kanüle, insbesondere eine Jamshidi Kanüle verwendet.

In einer weiteren Ausführungsform umfasst das Aufnahmemittel für das entnommene biologische Material ein oder mehrere Behältnisse, insbesondere drei Behältnisse, bevorzugt in der Form von Beuteln. Dies hat den Vorteil, dass nach der Entnahme des biologischen Materials eine Prozessierung des Materials im Aufnahmemittel selbst stattfinden kann, ohne dass eine Umfüllung des biologischen Materials nötig ist. Vor der Prozessierung kann das Aufnahmemittel mittels eines im Leitungsmittel befindlichen Konnektors vom Rest der Vorrichtung abgelöst werden. In einer alternativen Ausführungsform erfolgt das Ablösen mittels Abschweißen des zuführenden Leitungsmittels, wodurch ein zum Aufnahmemittel hinführender Abschnitt des Leitungsmittels gleichzeitig kontaminationsfrei verschlossen wird. Das Prozessieren kann insbesondere in der Zentrifugation bestehen, weshalb das Aufnahmemittel bevorzugt aus dafür geeignetem Material geformt ist.

Die im Vorratsmittel bevorratete Substanz ist bevorzugt eine gerinnungshemmende Substanz (ein Antikoagulanz), wie eine Heparin enthaltende Lösung oder eine CPD (Citrat, Phosphat, Dextrose)-Lösung.

Bevorzugter Weise ist die Vorrichtung als Einmalartikel (Wegwerfartikel) ausgestaltet, muss dann nach Benutzung nicht mehr gereinigt und erneut sterilisiert werden. Die Vorrichtung sollte aus einem Material gefertigt sein, dass sterilisierbar ist (etwa durch Bestrahlung mit gammaStrahlen oder durch chemische Sterilisierung).

Das der Erfindung zu Grunde liegende Problem wird auch durch die Verwendung einer hier beschriebenen Vorrichtung zur Entnahme einer biologischen Probe aus einem Organismus gelöst.

Ebenfalls gelöst wird das der Erfindung zu Grund liegende Problem durch ein Verfahren zur Entnahme biologischen Materials aus einem Organismus, umfassend das Entnehmen biologischen Materials aus einem Organismus mittels einer hier beschriebenen Vorrichtung.

Dieses Verfahren umfasst das Beschicken oder Befüllen des Entnahmemittels und/oder des Aufnahmemittels der Vorrichtung mit der im Vorratsmittel bevorrateten Substanz, insbesondere in Form eines Antikoagulanz. In weiteren Schritten wird ein Organismus mit dem Penetrationsmittel (etwa in Form einer Kanüle) penetriert, biologisches Material wird aus dem Organismus in das Entnahmemittel entnommen und das Material wird dann aus dem Entnahmemittel durch Erzeugung einer Druckdifferenz in das Aufnahmemittel überführt.

Das der Erfindung zu Grunde liegende Problem wird auch durch ein Verfahren zur Auftrennung einer biologischen Probe aus einem Organismus in mindestens zwei seiner Bestandteile gelöst. Dieses Verfahren umfasst das oben dargestellte Verfahren zur Entnahme einer biologischen Probe aus einem Organismus unter Verwendung der beschriebenen Vorrichtung. Darüber hinaus umfasst es den Schritt des Trennens des biologischen Materials im Aufnahmemittel in mindestens zwei seiner Bestandteile durch weitere Prozessierung, insbesondere durch Zentrifugation. So kann Blut z. B. in Serum und zelluläre Bestandteile aufgeteilt werden. Wenn das Aufnahmemittel der Vorrichtung verschiedene Behältnisse umfasst, kann in weiteren Schritten des Verfahrens z. B. das aus Blut abgetrennte Serum durch Ausüben von Druck auf das das Serum und die zellulären Bestandteile enthaltende Behältnis in eine weiteres Behältnis des Aufnahmemittels überführt werden.

In einer bevorzugten Ausführungsform betrifft die Erfindung keine Verfahren zur chirurgischen oder therapeutischen Behandlung des menschlichen oder tierischen Körpers und keine Diagnostizierverfahren, die am menschlichen oder tierischen Körper vorgenommen werden.

### Figuren

Es zeigen:
- Figur 1: eine Vorrichtung, bei der das Aufnahmemittel drei Beutel aufweist, die über ein zentrales Y-Stück miteinander verbunden sind, wobei sich ein Luer Lock un- terhalb des Y-Stücks befindet;
- Figur 2: eine Vorrichtung, bei der das Aufnahmemittel drei Beutel aufweist, die über ein zentrales Y-Stück miteinander verbunden sind, wobei sich ein 200 µm Filter unterhalb des Y-Stücks befindet;
- Figur 3: eine Vorrichtung, bei der das Aufnahmemittel einen Sammelbeutel und damit verbunden zwei Fraktionenbeutel aufweist;
- Figur 4: eine Vorrichtung, bei der das Aufnahmemittel einen Sammelbeutel und damit verbunden zwei Fraktionenbeutel aufweist, wobei ein 200 µm Filter im Lei- tungsmittel angeordnet ist;
- Figuren 5 bis 23: die Funktion der erfindungsgemäßen Vorrichtung und das Verfahren seiner Verwendung.

Die Zeichnungen der Figuren werden nachfolgend ausführlich beschrieben.

### Figur 1

Die gezeigte Entnahmevorrichtung 1 weist ein Entnahmemittel in Form einer (Kolben-) Spritze 2, ein Umleitungsmittel in Form eines Mehrwegehahns (hier Dreiwegehahns) 3, ein Aufnahmemittel 4 in Form dreier Beutel 4a, 4b, 4c und ein Leitungsmittel 5 in Form eines Schlauches auf.

Das Leitungsmittel 5 verbindet den Dreiwegehahn 3 zum einen lumig mit dem Aufnahmemittel 4 in Form dreier Beutel 4a, 4b, 4c und zum anderen lumig mit einem Vorratsmittel (nicht gezeigt). In der gezeigten Ausführungsform der Vorrichtung 1 ist das Entnahmemittel in Form einer Spritze 2 unmittelbar am Dreiwegehahn 3 angeordnet, was aber auch durch ein Leitungsmittel erfolgen kann.

Ein Penetrationsmittel im Form einer Jamshidi-Kanüle (nicht gezeigt) kann über ein Luer Lock am Dreiwegehahn 3 der Vorrichtung 1 angeschlossen werden. Ein (nicht gezeigtes) Vorratsmittel in Form eines Behältnisses für ein Antikoagulanz kann ebenfalls mit dem Leitungsmittel 5 der Vorrichtung 1 verbunden werden.

Die Spritze 2 ist mit dem Dreiwegehahn 3 verbunden und die gerinnungshemmende Substanz sowie die Beutel 4 sind durch das Leitungsmittel 5 fest miteinander verbunden, wodurch eine ständige Abnahme der alten Sprite und ein Ansetzen einer neuen Spritze entfällt. Als gerinnungshemmende Substanz kann beispielsweise unfraktioniertes Heparin verwendet werden, das für intravenöse (i.v.) Anwendungen geeignet ist.

Bei der Verwendung der Vorrichtung wird die (nicht gezeigte) Kanüle z. B. ins Knochenmark des Patienten eingeführt. Zunächst wird in die mit der Entnahmevorrichtung 1 verbundene Spritze 2 gerinnungshemmende Lösung aus dem Vorratsmittel angesaugt, welche mittels einem von zwei integrierten Adaptern (z. B. in Form eines Spike oder Female Luer) mit der Entnahmevorrichtung 1 verbunden wird und durch Integration eines ersten bakteriendichten Filters 11 gegenüber der Außenwelt funktionell geschlossen ist. Ein Eindringen von Keimen durch das Ansaugen der gerinnungshemmenden Lösung ist damit ausgeschlossen.

Durch die Spritze 2, die am Dreiwegehahn 3 befestigt ist, kann das biologische Material angesaugt werden und gelangt auf diese Weise in die Spritze 2. Ist die Spritze 2 befüllt, wird der Dreiwegehahn 3 umgelegt und damit die Verbindung zum Aufnahmemittel 4 geöffnet. Wird nun die Spritze 2 durch Druckausübung entleert, fließt das biologische Material in das Aufnahmemittel 4, ohne das geschlossene System der Vorrichtung 1 verlassen zu haben oder dass eine neue Spritze 2 hätte angeschlossen werden müssen. Durch Einbau von zwei Umschlagventilen 7, 8 in das Leitungsmittel 5 wird sichergestellt, dass das biologische Material nicht versehentlich in den falschen Beutel 4a, 4b, 4c überführt wird.

Der Vorgang der Entnahme biologischen Materials durch Befüllen und Entleeren des Entnahmemittels 2 kann beliebig wiederholt werden, ohne dass die Vorrichtung 1 an irgendeiner Stelle geöffnet werden müsste. Das Eindringen von gesundheitsschädlichen Keimen kann auf diese Weise verhindert werden. Lediglich die Verbindung zwischen Dreiwegehahn 3 und Jamshidi-Kanüle kann geöffnet werden, wenn der Punkteur mit Hilfe des Trocars eine andere Stelle in der harten Knochenschale durchbohren möchte.

Biologisches Material kann jede flüssige oder halbfeste biologische Substanz sein, wie Blut, Urin, Lymph- und andere Körperflüssigkeiten, Knochenmark oder Fett. Knochenmark ist bevorzugt.

Die Leitungsmittel 5 der erfindungsgemäßen Vorrichtung 1 sind vorzugsweise aus einem flexiblen Material. Bevorzugt werden Schläuche verwendet mit einem Außendurchmesser von 3 mm bis 6 mm, bevorzugt 4,1 mm und mit einem Innendurchmesser von 1,5 mm bis 4,5 mm, bevorzugt 2,5 mm und einer Spannweite von 0,5 mm bis 10 mm, vorzugsweise 2 mm bis 5 mm. Bevorzugte Materialien sind beispielsweise Kunststoff, Polyethylen und/ oder Materialien, die durchsichtig und/oder farblos und ungiftig sind.

Die Bestandteile des Entnahmemittels 3 müssen dafür geeignet sein, biologisches Material entweder manuell oder maschinell anzusaugen, aufzufangen und wieder freigeben zu können. Vorzugsweise kann dies eine Spritze 2 mit 2 ml bis 20 ml, vorzugsweise 10 ml Volumen sowie einbis mehrfacher vorzugsweise doppelter Gummidichtung sein, wie z. B. Polypropylen Spritzen mit Polyisopren Dichtung und Luer Lock Anschluss. Die erfindungsgemäße feste Verbindung zwischen dem Entnahmemittel 2 für das biologische Material und dem Dreiwegehahn 3 verhindert das Eindringen von partikelbehafteter Luft sowie gesundheitsschädlichen Keimen.

In der gezeigten Ausführungsfrom weist die Vorrichtung 1 zwei Zugänge zum Leitungsmittel 5 auf, nämlich einen Spike 9 und einen weiblichen Luer-Konnektor 10 auf. Die Bestandteile eines ersten Zugangs für das Vorratsmittel (z. B. in Form eines Infusionsbeutels), welcher zum Dreiwegehahn 3 führt, der mit dem Entnahmemittel 2 verbunden ist, umfassen in der gezeigten Ausführungsform einen Spike 9 mit einem Innendurchmesser von 1 mm bis 5 mm, vorzugsweise 3 mm, und einem Außendurchmesser von 2 mm bis 8 mm, vorzugsweise 4,1 mm bzw. passend für die verwendete Schlauchgröße zum Verbinden mit einem Infusionsbeutel, sowie einen weiblichen Luer-Konnektor 10 (bevorzugt aus Acryl mit einem Durchmesser von 3,1 mm bis 3,4 mm (Innendurchmesser) bzw. passend zur verwendeten Schlauchgröße) zum Verbinden mit einer Spritze 2, einen ersten Filter 11 zur Verhinderung des Eindringens von Bakterien über die angesaugte Flüssigkeit, sowie ein erstes Rückschlagventil 7, so dass die Fließrichtung in Richtung zum Dreiwegehahn 3 hin festgelegt ist und kein biologisches Material in Richtung Spike 9 und/oder weiblicher Luer-Konnektor 10 zurückfließen kann.

Der erste Filter 11 hat eine Porengröße von 0,05 µm bis 0,2 µm, bevorzugt von 0,2 µm. Bevorzugt kann der Filter 11 gesundheitsschädliche Keime wie Bakterien, Pilze und Partikel usw. auffangen, so dass eine Kontamination der Vorrichtung 1 vermieden wird. Weiter ist der Filter 11 bevorzugt so ausgestaltet, dass er ein Selbstpriming (Benetzung der Oberfläche mit Flüssigkeit und Verdrängung der Luft im Gehäuse) durchführen kann. Weiter bevorzugt hat er hohe Flussraten (mehr als 5 ml pro Minute bis 10 ml pro Minute, bevorzugt 10 ml pro Minute).

Das erste (Rückschlag-) Ventil 7 ist vorzugsweise zwischen Bakterienfilter 11 und Umleitungsmittel z.B. in Form eines Dreiwegehahns 3 angeordnet. Das Ventil 7 erspart der anwendenden Person das Umlegen eines Dreiwegehahns 3, so dass die Anwendung besonders einfach ist im Gegensatz zu den oben beschriebenen aus dem Stand der Technik bekannten Verfahrensweisen, die ein ständiges An- und Absetzen und Neumontieren der Spritze erfordern bzw. bei Verwendung eines weiteren Dreiwegehahnes ein höheres Risiko für Anwendungsfehler beherbergen.

Das erste (Rückschlag-) Ventil 7 hat vorzugsweise einen hohen Öffnungsdruck von 0,2 - 5 PSI (Pascal per square inch) (14 - 345 mbar) vorzugsweise 1,5 PSI (103 mbar) (z. B. Qosina Checkvalve, Durchmesser 0,130 - 0,138 (Außendurchmesser), Öffnungsdruck (cracking pressure) 0,5 - 5,0 PSI (34 - 340 mbar), vorzugsweise 1-2 PSI (69 - 138 mbar), weiter vorzugsweise 1,5 PSI (103 mbar) (Gehäuse Acryl, Ventil aus Silikon), um das versehentliche Überfließen der Inhaltsstoffe aus dem angeschlossenen Antikoagulanz bei erhöhter Lagerung beispielsweise an einen Infusionständer durch das Ventil zu verhindern. Öffnet sich das Ventil 7 bereits bei geringem Druck, fließt das Antikoagulanz von selbst in die Auffangbehältnisse. Dies wird durch den erhöhten Öffnungsdruck verhindert.

Der Spike 9 wird bevorzugt mit dem Vorratsmittel mit einer fertigen Lösung aus CPD (Citrat, Phosphat und Dextrose) verbunden, vorzugsweise mit einem Volumen von CPD 250 ml, enthaltend Citrat, Phosphat und Dextrose.

In der gezeigten Ausführungsform kann der weibliche Luer-Konnektor 10 mit einer Spritze 2 mit männlichem Luer-Anschluss verbunden werden, welche ein Gemisch enthaltend gerinnungshemmende Substanzen vorzugsweise Heparin (z. B. Heparin unfraktioniert, für i.v. Anwendungen geeignet, 5000 IE/ml,) und entweder NaCl 0,9 % oder CPD beinhaltet. Die Spritze 2 weist bevorzugt ein Volumen von 10 ml bis 50 ml auf.

Ein weiterer am Dreiwegehahn 3 angeordneter Zugang umfasst eine Verbindung zum Aufnahmemittel 4. Eine Strecke von 2 cm bis 5 cm Schlauch 5, bevorzugte Länge 3 cm bei einem Innendurchmesser von 2,5 mm, wird zwischen dem Dreiwegehahn 3 und einem zweiarmigen Y-Stück der Aufteilung der beiden zu- und abführenden Schläuche integriert, um die Flexibilität bzw. Handhabbarkeit der Ansaugvorrichtung (Dreiwegehahn 3 plus Spritze 2) zu verbessern. Die Länge des Schlauchsegmentes des Leitungsmittels 5 ist so zu bemessen, dass die pro Zyklus angesaugte Menge an Antikoagulanz mindestens doppelt so groß ist wie das im Schlauchsegment befindliche Totvolumen. In einer bevorzugten Ausführung beträgt das Totvolumen des Schlauchsegmentes 0,5 ml bei einer Entnahme von jeweils 1 ml gerinnungshemmender Substanz.

In einer bevorzugten Ausführung umfasst das Schlauchsegment des Leitungssystems 5 zwischen Aufnahmemittel 4 und Dreiwegehahn 3 ein zweites Rückschlagventil 8 ("duckbill check valve") sowie ein Luer lock Konnektor, mittels dessen eine Verbindung zu einem Abschnitt des Leitungssystems 5 herstellbar ist, die mit dem Aufnahmemittel 4 (hier in Form dreier miteinander verbundener Auffangbehältnisse 4a, 4b, 4c) verbunden ist. Die Behältnisse 4a, 4b, 4c sind vorzugsweise Beutel, die bevorzugt aus Kunststoff wie PVC bestehen. Das Volumen der Beutel kann 100 ml bis 1000 ml, bevorzugt 300 ml betragen.

Das Duckbill check valve 8 besteht beispielsweise aus Polycarbonat (Gehäuse) und Silikon (Ventil), und ermöglicht durch seine Form die Passage von Knochensplittern, ohne seine Schließfähigkeit zu verlieren, mit einer Größe passend zum Verbindungs- bzw. Schlauchsystem vorzugsweise 0,130 Zoll (Außendurchmesser), mit einem Öffnungsdruck von 0,05 bis 0,5 PSI (3,4 - 34 mbar), bevorzugt 0,112 PSI (7,7 mbar) (0,05 bis 0,5 PSI tolerabler Bereich). Die beiden Ventile 7, 8 im Leitungsmittel 5 müssen derartig konfiguriert, also aufeinander abgestimmt sein, dass das zweite Ventil 8 sich nicht dadurch öffnet, dass man an der Spritze 2 über das erste Ventil 7 zieht bzw. umgekehrt: wenn die Spritze 2 nach dem Aufziehen wieder in das System über das zweite Ventil 8 entleert wird, darf sich das erste Ventil 7 nicht öffnen. Dies wird durch die erfindungungsgemäße Vorrichtung 1 sichergestellt.

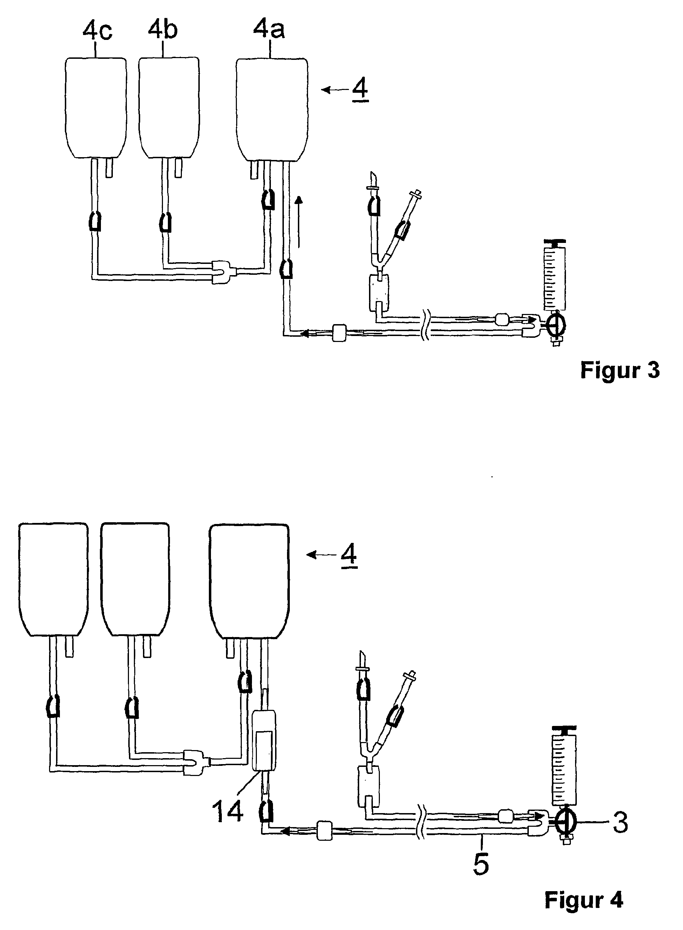

Der Luer Lock Konnektor ist zwischen dem zweiten Rückschlagventil 8 und den Behältnissen 4a, 4b, 4c angeordnet und ermöglicht dem Anwender das Abtrennen des Aufnahmemittels 4 vom übrigen Teil der Vorrichtung 1, sowie die Ausgestaltung des Aufnahmemittels 4 entweder in Form eines ersten Auffangbehälters 4a, der mit weiteren Auffangbehältern verbunden ist 4b, 4c (vgl. Figuren 3 und 4) oder in Form dreier Behältnisse 4a, 4b, 4c (wie hier gezeigt). Der männliche Luer Lock Konnektor hat bevorzugt einen Durchmesser von 2 mm bis 4 mm, bevorzugt 3,1 mm bis 3,4 mm (Innendurchmesser) aus Acryl bzw. passend zum verwendeten Schlauch. Der männliche Konnektor hat einen I.D. 3,1 mm bis 3,4 mm bzw. passend für verwendeten Schlauch und besteht vorzugsweise aus Acryl.

In der gezeigten Ausführungsform weist das Leitungsmittel 5 zwischen dem Umleitungsmittel 3 und dem Aufnahmemittel 4 ein dreiarmiges Y-Stück auf, so dass sich das Leitungsmittel 5 in dem gezeigten Beispiel der Vorrichtung in drei Äste verzweigt, wobei ein Ast jeweils in einem Beutel 4a, 4b, 4c mündet.

Bevorzugt werden an den Aufnahmebeuteln 4a, 4b, 4c Spike-Anschlüsse verwendet, um das Zuführen oder Entnehmen von Flüssigkeit in bzw. aus allen Aufnahmebeuteln 4a, 4b, 4c zu ermöglichen oder um durch Verbindung mit weiteren Aufnahmebeuteln 4a, 4b, 4c die Flexibilität der Auftrennung der Fraktionen zu erhöhen. Im Einzelnen kann dies die Zugabe von Medikamenten oder Substanzen sein, die einen Einfluss auf die Bestandteile der Körperflüssigkeit haben. Ferner können Proben aus der Körperflüssigkeit vor und nach der Auftrennung in Fraktionen mittels Zentrifugation gewonnen werden. Durch die Verbindung mehrerer Auffangbeutel ist es möglich, die Körperflüssigkeit in mehrere Fraktionen aufzutrennen und/oder in mehreren Behältnissen getrennt weiter zu prozessieren.

Die Auffangbehältnisse (Beutel) des Aufnahmemittels 4 sind bevorzugt so beschaffen, dass die Körperflüssigkeit mittels Zentrifugation in einzelne Fraktionen auftrennbar ist. Bevorzugt sind dazu Beutel aus DEPC freiem Kunststoff.

Die zu den Beuteln 4a, 4b, 4c des Aufnahmemittel führenden Leitungsmittel 5 können jeweils mit einer Klemme 15 versehen sein, um das Auslaufen und das Überführen von Flüssigkeit nach der Zentrifugation manuell zu steuern.

Als Köperflüssigkeit kommen folgende Flüssigkeiten in Frage: Knochenmark, wobei kleine Knochensplitter und Bröckel optional durch Filtration entfernt werden können; Blut, wobei in der Regel eine Filtration lediglich vor Rückgabe in den Körper notwendig sein kann; Fettgewebe, wobei die Schläuche, Dreiwegehahn, Filter und Ventile derart gestaltet sein sollen, dass es nicht zu einer Verstopfung durch Fettvakuolen kommt.

Die gesamte Vorrichtung 1 ist bevorzugt sterilisierbar, um die Möglichkeit zu schaffen, die entnommene Körperflüssigkeit oder einzelne Bestandteile frei von Krankheitserregern weiterzuverarbeiten oder wieder in den Körper zu injizieren.

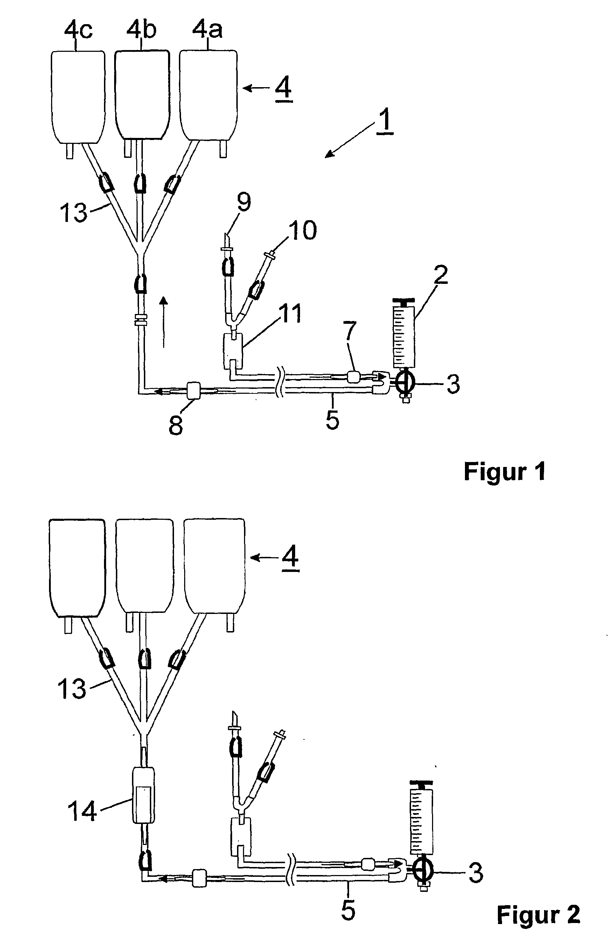

Die in den Figuren 2 bis 4 gezeigten Ausführungsformen entsprechen weitestgehend der oben dargestellten Ausführungsform, so dass im Folgenden nur die Abweichungen gegenüber der eben beschriebenen Ausführungsform dargestellt werden.

### Figur 2

In der gezeigten Ausführungsform ist im Leitungsmittel 5 zwischen Dreiwegehahn 3 und Aufnahmemittel 4 ein zweiter Filter 14 angeordnet, der bevorzugt ein Porengröße von 40 µm bis 500 µm, vorzugsweise von 150 µm bis 220 µm, weiter bevorzugt von 200 µm aufweist.

Der zweite Filter 14 dient zum Abfiltern von Bröckeln, Klumpen, Zellhaufen, Blutfetten, Knochenteilen bzw. -splittern und anderen Bestandteilen. Die Filtration ist sinnvoll, wenn die Körperflüssigkeit ohne Abtrennung der größeren Partikel wieder in einen Körper eingebracht werden soll. Der zweite Filter 14 sollte dann verwendet werden, wenn das entnommene und in das Aufnahmemittel 4 überführte biologische Material weiter prozessiert werden soll, etwa durch Zentrifugation.

In der gezeigten Ausführungsform weist der erste Beutel 4a des Aufnahmemittels 4 ein Leitungsmittel 5 zum Abfluss von biologischem Material aus dem ersten Beutel in mindestens einen weiteren, hier in zwei weitere Beutel 4b, 4c auf. Das abführende Leitungsmittel 5, welches aus dem Ausgang der ersten Aufnahmebeutels 4a des Aufnahmemittels 4 kommt, ist mit einem Y-Stück 13 verbunden, so dass sich das Leitungsmittel verzweigt und ein Ast des Leitungsmittels 5 zum zweiten Beutel 4b, und der andere Ast des Leitungsmittels 5 zum zweiten Beutel 4c führt. Andere Ausführungsformen mit einer praktisch beliebigen Zahl von Beuteln sind möglich.

### Figur 3

Die gezeigte Ausführung der Vorrichtung 1 umfasst ein Aufnahmemittel 4 mit einem ersten Auffangbeutel 4a des Aufnahmemittels 4, der seinerseits mit mindestens einem, in dem gezeigten Beispiel mit zwei weiteren Auffangbeuteln (einem zweiten Beutel 4b und dritten Beutel 4c) lumig verbunden ist. Bei dieser Ausführung ist sichergestellt, dass die im Schlauchabschnitt zum ersten Beutel 4a des Aufnahmemittels 4 befindliche Körperflüssigkeit nach einer Auftrennung der Fraktionen mit den getrennten Fraktionen in dem zweiten Beutel 4b und dritten Beutel 4c nicht vermischt werden kann.

Bei dieser Ausführungsform ist einerseits die Filtration von Partikeln aus der Körperflüssigkeit und andererseits die Verhinderung der Durchmischung zwischen gesammelter und aufgetrennter Körperflüssigkeit gegeben.

### Figur 4

Die gezeigt weitere Ausführung der Vorrichtung 1 entspricht der in Figur 3 gezeigten, und weist weiterhin einen zweiten Filter 14 auf, der im Leitungsmittel 5 zwischen dem Aufnahmemittel 4 und dem Umleitungsmittel 3 angeordnet ist. Dieser zweite Filter 14 ist bereits im Zusammenhang mit Figur 2 beschrieben worden.

### Figuren 5 bis 23

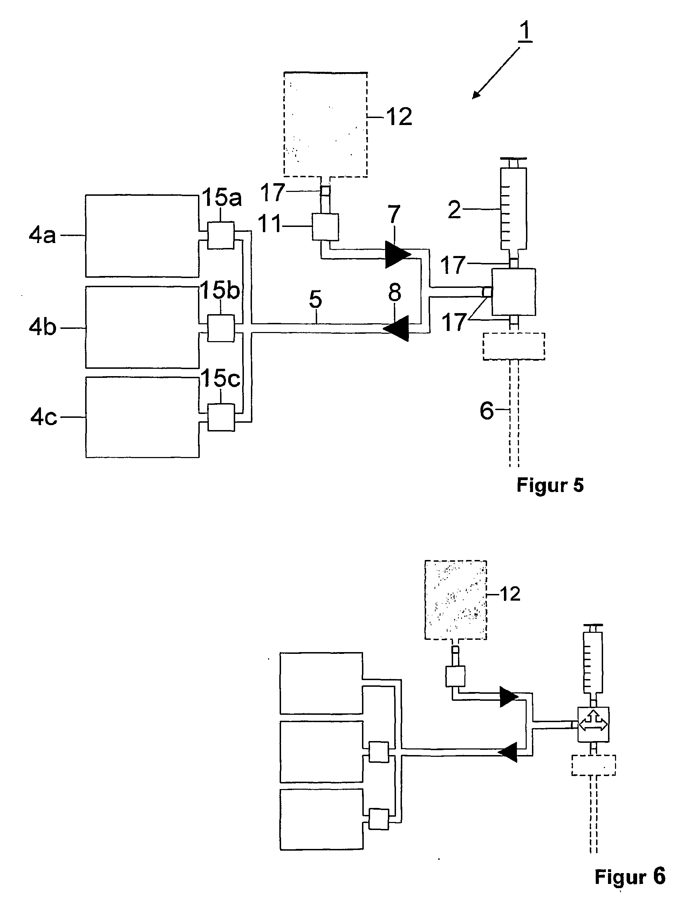

In den Figuren 5 bis 23 wird die Vorrichtung 1 und ihre Funktionsweise dargestellt.

Wie in Figur 5 gezeigt weist die Vorrichtung 1 ein Umleitungsmittel 3 in Form eines Dreiwegehahns auf, das seinerseits drei Anschlussmittel 17 in Form eines Luer Locks oder eines Spikes aufweist. Der Dreiwegehahn ist mittels eines ersten Anschlussmittels 17 an ein Entnahmemittel 2 in Form einer Spritze lumig angebunden. Mittels eines zweiten Anschlussmittels 17 ist der Dreiwegehahn lumig mit einem Penetrationsmittel 6 in Form einer Jamshidi-Kanüle verbunden, und mittels eines dritten Anschlussmittels 17 mit einem Leitungsmittel 5 in Form eines flexiblen Schlauches verbunden. Der Schlauch 5 weist eine erste Verzweigung auf, wobei ein erster Ast des Schlauches 5 mit einem Vorratsmittel 12 in Form eines Antikoagulanz enthaltenden Infusionsbeutels verbunden ist. Der andere Ast des Schlauches 5 führt zu einem Aufnahmemittel 4 in Form dreier Beutel 4a, 4b, 4c, wobei das Leitungsmittel eine Verzweigung der Art aufweist, dass zu jedem Beutel ein einzelner Ast des Schlauches 5 führt. In einem Abschnitt des Schlauches 5, kurz vor der Einmündung des Schlauches in den Beutel 4a, 4b, 4c ist jeweils eine Klemme 15a, 15b, 15c angeordnet, so dass die Zuleitung des Schlauches 5 in den Beutel 4a, 4b, 4c verschlossen werden kann.

In der in der Figur 6 dargestellten Zeichnung befindet sich die Vorrichtung 1 in einer funktionsbreiten Position. Dabei ist insbesondere das Vorratsmittel 12 mit einem Antikoagulanz befüllt, so dass das zu entnehmende biologische Material, insbesondere Knochmark, vor der Gerinnung bewahrt werden kann.

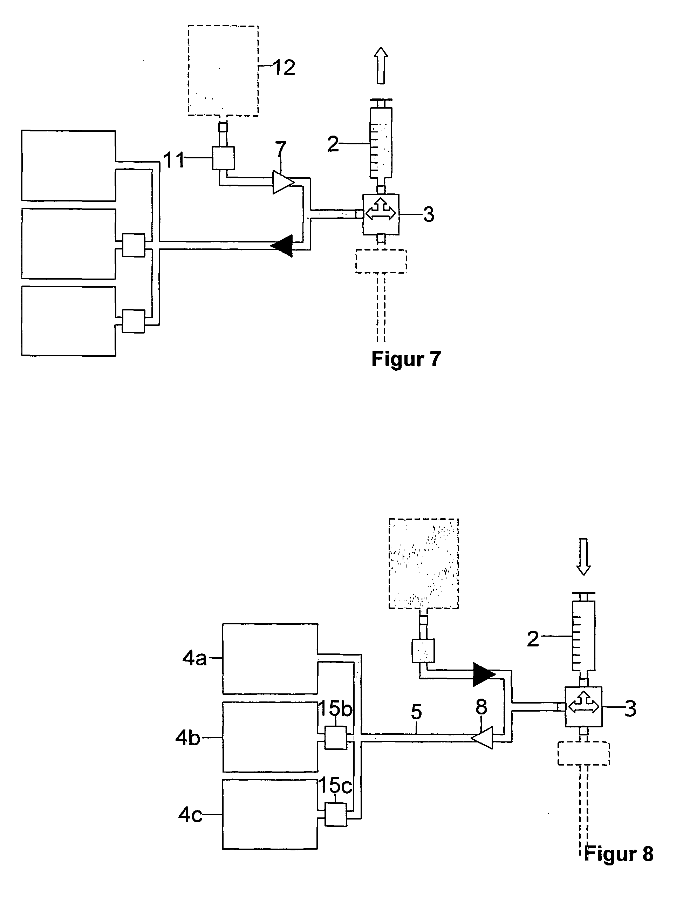

Bei der Verwendung der Vorrichtung 1 ist der Dreiwegehahn 3 zunächst in einer ersten Funktionsstellung, bei der das Vorratsmittel lumig mit der Spritze 2 verbunden ist. Durch das Herausziehen des Stempels der Spritze 2 entsteht in der Spritze 2 ein Unterdruck, so dass das Antikoagulanz aus dem Infusionsbeutel 12 durch das Leitungsmittel 5 über den Dreiwegehahn 3 in die Spritze 2 fließt. Dazu ist zwischen dem Infusionsbeutel 12 und der Spritze 2 im Leitungsmittel ein erster Filter 11 angeordnet, der dem Herausfiltern von stückigem Material oder Mikroorganismen dient. Weiterhin weist der Schlauch 5 ein erstes Umschlagventil 7 auf, das die Fließrichtung des Antikoagulanz von dem Infusionsbeutel 12 in Richtung zum Dreiwegehahn 3 vorgibt (Figur 7). Ein geöffnetes Ventil ist in den Figuren als weißes Dreieck dargestellt, während ein geschlossenes Ventil schwarz dargestellt ist.

In dem zu den Beuteln 4a, 4b, 4c führenden Ast des Schlauches 5 ist ein zweites Umschlagventil 8 angeordnet, das beim Hereindrücken des Stempels in die Spritze 2 bei unveränderter Position des Dreiwegehahns 3 das gerinnungshemmende Mittel im Schlauch 5 in Richtung der Beutel 4a, 4b, 4c passieren läßt. In dem hier gezeigten Beispiel sind der zweite Beutel 4b und der dritte Beutel 4c jeweils durch eine zweite Klemme 15b und eine dritte Klemme 15c abgeklemmt, so dass das Antikoagulanz nur den ersten Beutel 4a fließen kann (Figur 8).

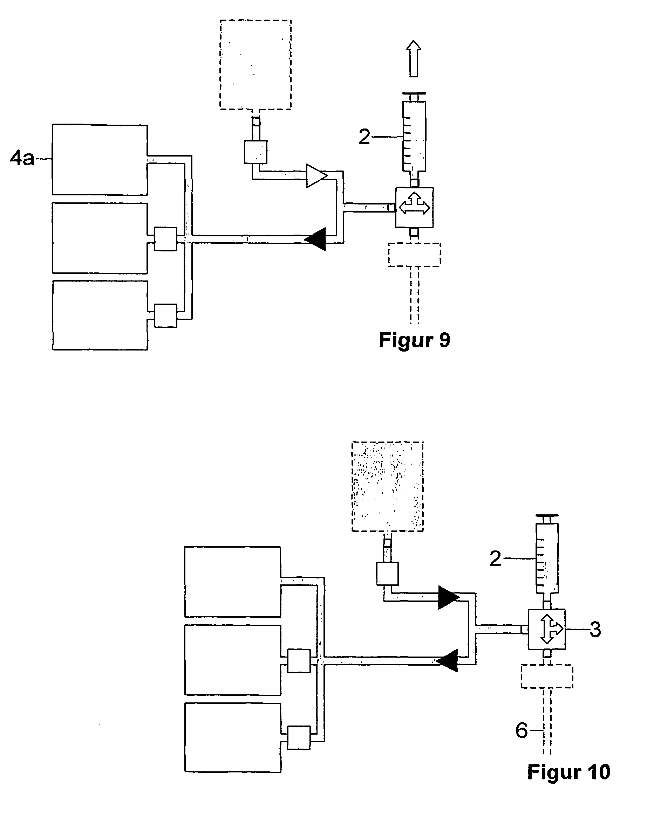

Sofern das Volumen der Spritze 2 zur Befüllung des ersten Beutels 4a nicht ausreicht, kann die Spritze 2 durch nochmaliges Herausziehen des Stempels erneut mit dem Antikoagulanz befüllt werden. In dem hier gezeigten Beispiel wird Antikoagulanz in die Spritze 2 vorgelegt (Figur 9).

Nun kann die Kanüle 6 in die vorgesehene anatomische Struktur des Organismus eingeführt werden, um mit der Entnahme des gewünschten biologischen Materials zu beginnen. Dazu wird der Dreiwegehahn 3 in eine weitere Funktionsstellung gebracht, bei der die Spritze 2 lumig mit der Kanüle 6 verbunden ist (Figur 10).

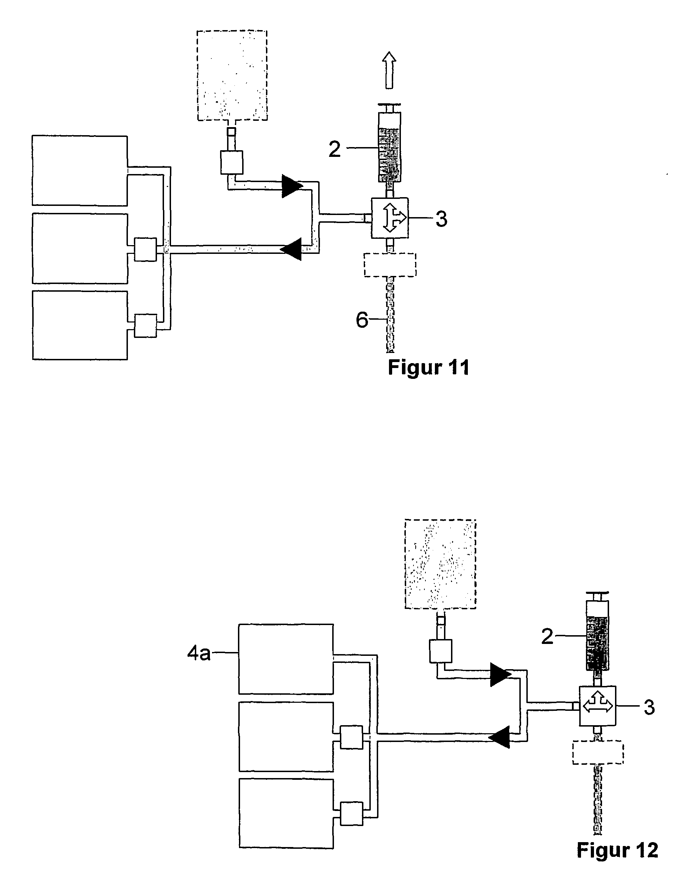

Durch weiteres Herausziehen des Stempels der Spritze 2 wird eine Druckdifferenz in Bezug auf das Zielgewebe aufgebaut, so dass das zu entnehmende biologische Material durch die Spritze 6 über den Dreiwegehahn 3 in die Spritze 2 fließt (Figur 11).

Nachdem die Spritze 2 mit biologischen Material befüllt worden ist, wird der Dreiwegehahn 3 in die erste Funktionsstellung verbracht, bei der die Spritze 2 lumig mit dem Infusionsbeutel 12 und dem Beutel 4a verbunden ist (Figur 12).

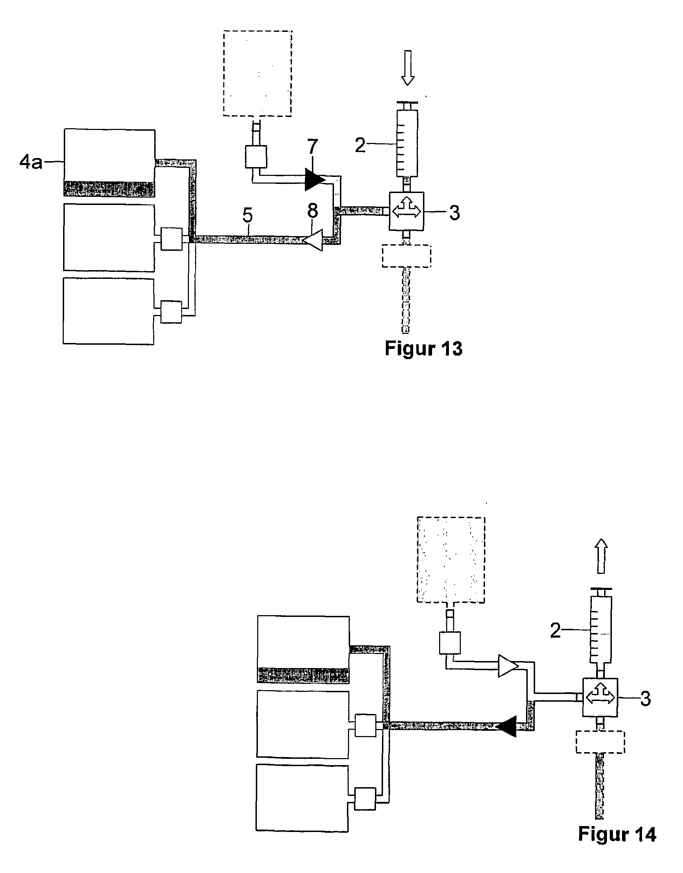

Durch Herunterdrücken des Stempels der Spritze 2 fließt das biologische Material aus der Spritze 2 über den Dreiwegehahn 3 und den Schlauch 5 in den ersten Beutel 4a. Das erste Umschlagventil 7 und das zweite Umschlagventil 8 im Schlauch 5 verhindern dabei, dass das entnommene biologische Material in den Infusionsbeutel 12 fließen kann (Figur 13).

Aufgrund des ersten Umschlagventils 7 und des zweiten Umschlagventils 8 ist es ebenfalls möglich, dass die Spritze 2 durch Herausziehen des Stempels in einem nächsten Schritt ohne Veränderung der Position des Dreiwegehahns 3 erneut mit Antikoagulanz befüllt werden kann (Figur 14).

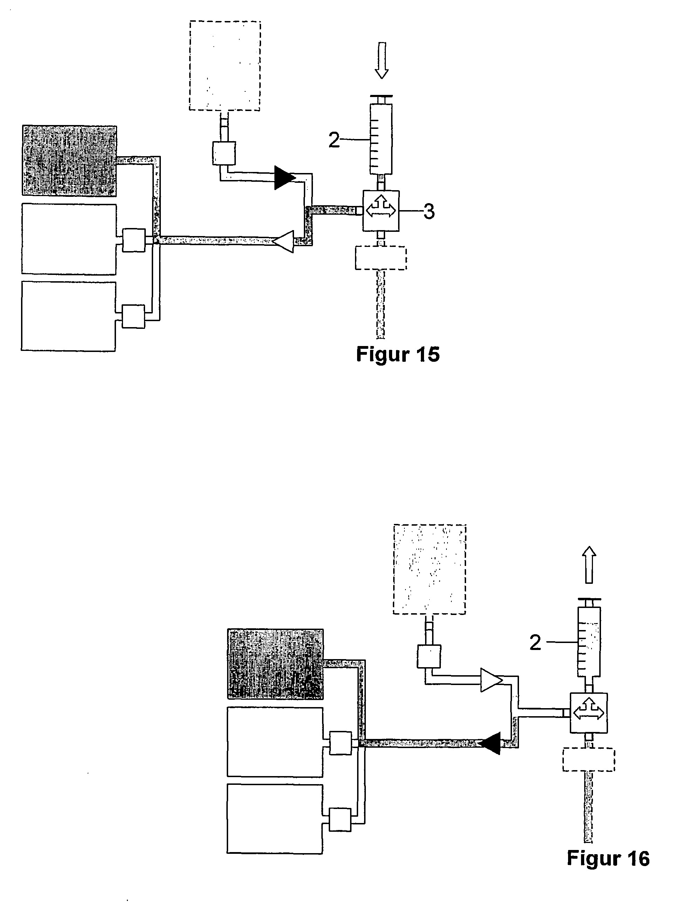

Nach Befüllung der Spritze 2 mit Antikoagulanz kann dieses gerinnungshemmende Mittel durch Herausdrücken aus der Spritze 2 ohne Verstellung der Position des Dreiwegehahns 3 in den ersten Beutel 4a befördert werden (Figur 15).

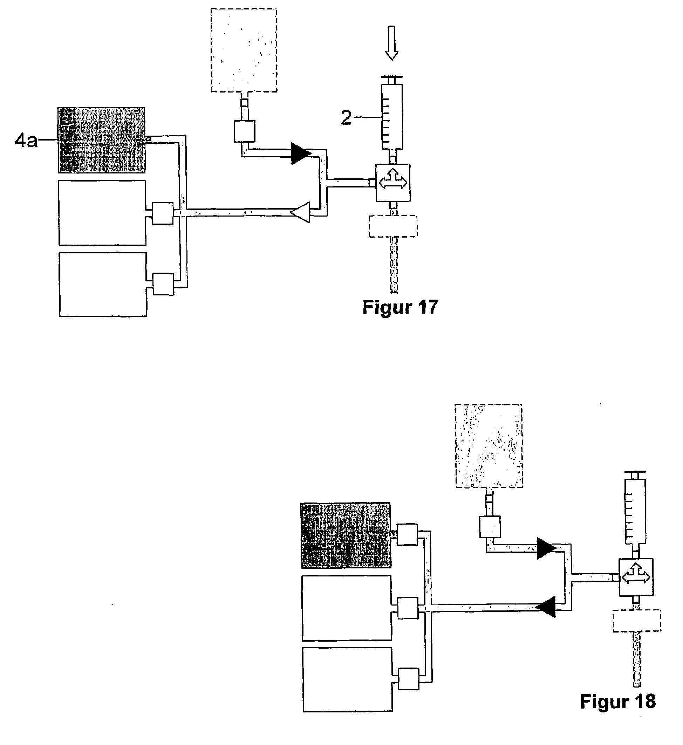

In Figur 16 ist die abermalige Befüllung der Spritze 2 mit Antikoagulanz gezeigt. Figur 17 zeigt die Überleitung des Antikoagulanz in den ersten Beutel 4a, wie oben beschrieben. Nach der Entnahme des biologischen Materials aus dem Organismus kann sich die Vorrichtung 1 somit in der in der Figur 18 gezeigten Position befinden.

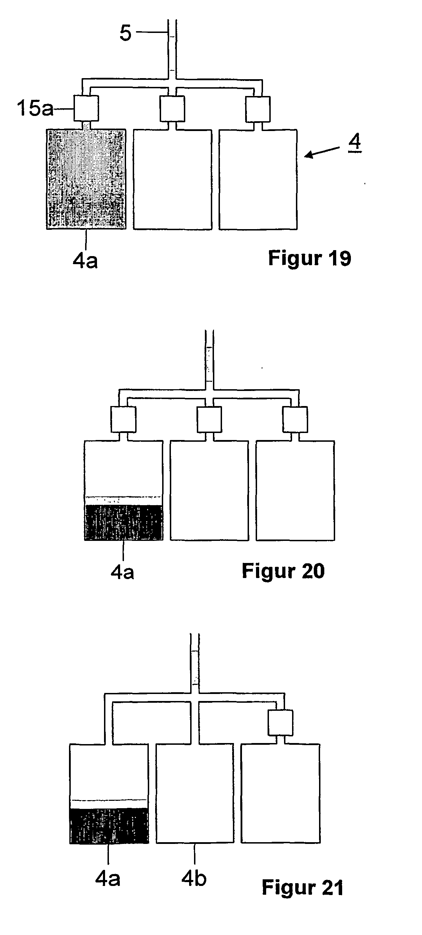

Zur weiteren Prozessierung des entnommenen biologischen Materials kann das Aufnahmemittel 3 beispielsweise durch Abschweißen des Schlauches 5 an einer geeigneten Stelle erfolgen. Der Schlauchzulauf 5 zum ersten Beutel 4a wird vor der weiteren Prozessierung des entnommenen biologischen Materials mit einer ersten Klemme 15a verschlossen (Figur 19).

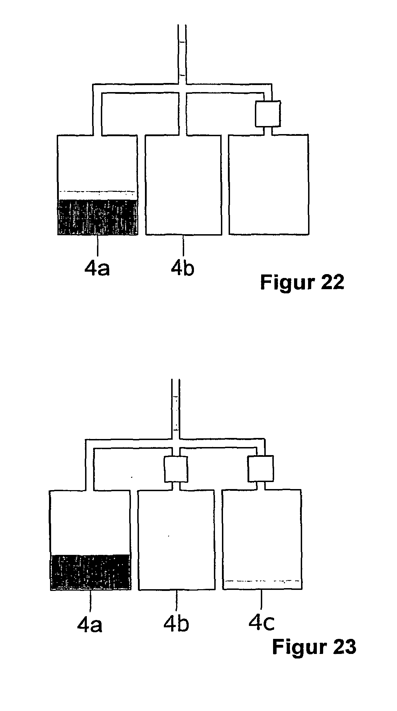

Das Aufnahmemittel 4 der Vorrichtung 1 kann nach seiner Abtrennung beispielsweise einer Zentrifugation unterworfen werden, wobei beispielsweise zelluläre von flüssigen Bestandteilen der biologischen Probe voneinander getrennt werden können. Diese Komponenten des biologischen Materials sind in Figur 20 im ersten Beutel 4a durch verschiedene Grauschattierungen dargestellt. Zur Trennung der Komponenten des biologischen Materials werden in dem hier gezeigten Beispiel die erste Klemme 15a und die zweite Klemme 15b geöffnet, so dass der erste Beutel 4a mit dem zweiten Beutel 4b über den Schlauch 5 lumig verbunden ist (Figur 21). Durch Druckausübung auf den ersten Beutel 4a kann die oben angeordnete Komponente des biologischen Materials mittels des Schlauches 5 aus dem ersten Beutel 4a in den zweiten Beutel 4b überführt werden (Figur 22). Die Auftrennung des biologischen Materials kann mittels verschiedener Beute 4a, 4b, 4c erfolgen, und kann auch durch mehrmalige Prozessierung, beispielsweise durch mehrfache Zentrifugation erfolgen (Figur 23).

Die einzelnen Beutel 4a, 4b, 4c können nun beispielsweise durch Abschweißen von den übrigen Bestandteilen in der Vorrichtung getrennt werden oder das aufgetrennte biologische Material kann mittels Punktion der Beutel, etwa mit einer Kanüle entnommen werden.

### Bezugszeichenliste

- 1: Vorrichtung (Entnahmevorrichtung)
- 2: Entnahmemittel
- 3: Umleitungsmittel
- 4: Aufnahmemittel
- 5: Leitungsmittel
- 6: Penetrationsmittel
- 7: erstes Umschlagventil
- 8: zweites Umschlagventil
- 9: Spike
- 10: weiblicher Luer-Konnektor
- 11: erster Filter
- 12: Vorratsmittel
- 13: Y-Stück, dreiarmig
- 14: zweiter Filter
- 15: Klemme
- 17: Anschlussmittel

## Patentansprüche

1. Vorrichtung (1) zur Entnahme biologischen Materials aus einem Organismus, **gekennzeichnet dadurch, daß** die Vorrichtung
ein Umleitungsmittel (3) aufweist, zur wahlweisen lumigen Verbindung eines Entnahmemittels (2) zur Entnahme biologischen Materials aus einem Organismus, mit
- einem Penetrationsmittel (6) zur Penetration in den Organismus,
- einem Aufnahmemittel (4) zur Aufnahme des Materials aus dem Organismus, und
- einem Vorratsmittel (12) zur Bevorratung einer Substanz,
wobei das Umleitungsmittel (3) derart ausgestaltet ist, dass das Entnahmemittel (2)
- mit dem Vorratsmittel (12) und mit dem Aufnahmemittel (4), oder
- mit dem Penetrationsmittel (6),
lumig verbindbar ist.

2. Vorrichtung nach Anspruch 1, **dadurch gekennzeichnet, dass** die lumige Verbindung zumindest zum Teil durch ein Leitungsmittel (5) verwirklicht ist.

3. Vorrichtung nach Anspruch 1 oder 2, wobei das Umleitungsmittel (3) zwischen dem Entnahmemittel (2) und dem Penetrationsmittel (6) angeordnet ist.

4. Vorrichtung nach Anspruch 1 bis 3, wobei das Umleitungsmittel (3) zwischen dem Vorratsmittel (12) und dem Aufnahmemittel (4) angeordnet ist.

5. Vorrichtung nach Anspruch 1 bis 4, wobei zwischen dem Vorratsmittel (12) und dem Umleitungsmittel (3) ein erstes Ventil (7) im Leitungsmittel (5) angeordnet ist, welches derart konfiguriert ist, dass das biologische Material nur vom Vorratsmittel (12) in Richtung Umleitungsmittel (3) fließen kann.

6. Vorrichtung nach Anspruch 1 bis 5, wobei zwischen dem Umleitungsmittel (3) und dem Aufnahmemittel (4) ein zweites Ventil (8) im Leitungsmittel (5) angeordnet ist, welches derart konfiguriert ist, dass das biologische Material nur vom Umleitungsmittel (3) in Richtung Aufnahmemittel (4) fließen kann.

7. Vorrichtung nach Anspruch 1 bis 6, wobei im Leitungsmittel (5) ein Filter (11, 14) zum Filtern von Mikroorganismen und/oder stückigem biologischen Material angeordnet ist.

8. Vorrichtung nach Anspruch 1 bis 7, wobei das Entnahmemittel (2) als Spritze ausgeformt ist.

9. Vorrichtung nach Anspruch 1 bis 8, wobei das Umleitungsmittel (2) als Mehrwegehahn, insbesondere als Dreiwegehahn ausgeformt ist.

10. Vorrichtung nach Anspruch 1 bis 9, wobei das Leitungsmittel (5) als Schlauch ausgeformt ist.

11. Vorrichtung nach Anspruch 1 bis 10, wobei das Penetrationsmittel (6) als Kanüle ausgeformt ist.

12. Vorrichtung nach Anspruch 1 bis 11, wobei das Aufnahmemittel (4) für das biologische Material ein oder mehrere Behältnisse umfasst.

13. Vorrichtung nach Anspruch 1 bis 12, wobei das Aufnahmemittel (4) derart ausgestaltet ist, dass das biologische Material im Aufnahmemittel (4) in mindestens zwei seiner Bestandteile auftrennbar ist, insbesondere aus flexiblem, zentrifugierbarem, durchsichtigem oder sterilisierbarem Material besteht.

14. Vorrichtung nach Anspruch 1 bis 13, wobei die im Vorratsmittel (12) bevorratete Substanz ein Antikoagulanz ist.

15. Vorrichtung nach Anspruch 1 bis 14, wobei die Vorrichtung als Einmalartikel ausgestaltet ist und/oder sterilisierbar ist.

## Claims

1. Device (1) for removal of biological material from an organism, comprising
- a redirection means (3) for selecting luminal connection of a removal means (2) for removing biological material from an organism with
- a penetration means (6) for penetrating into the organism,
- a reception means (4) for receiving the material froth the organism, and
- a reservoir means (12) for storage of a substance,
wherein the redirection means (3) is configured such that the removal means (2) can form a luminal connection with
- the reservoir means (12) and with the reception means (4), or
- with the penetration means (6).

2. Device (1) according to claim 1, **characterized in that** the luminal connection is realized at least in part by a conduit means (5).

3. Device (1) according to claims 1 and 2 wherein the redirection means (3) is located between the removal means (2) and penetration means (6).

4. Device (1) according to claims 1 to 3, wherein the redirection means (3) is located between the reservoir means (12) and the reception means (4).

5. Device (1) according to claims 1 to 4, wherein a first valve (7) is located in the conduit means (5) between the reservoir means (12) and the redirection means (3), wherein the first valve (7) is configured such that the biological material can only flow from the reservoir means (12) towards the redirection means (3).

6. Device (1) according to claims 1 to 5, when a second valve (8) is located in the conduit means (5) between the redirection (3) and the reception means (4), wherein the second valve (8) is configured such that the biological material can only flow in the direction from the redirection means (3) towards the reception means (4).

7. Device (1) according to claims 1 to 6, wherein a filter (11, 14) for filtering microorganisms and/or pieces of biological material is located in the conduit means (5).

8. Device (1) according to claims 1 to 7, wherein the removal means (2) is a syringe.

9. Device (1) according to claims I to 8, wherein the redirection means (3) is a multi-way cock, in particular a three-way cock.

10. Device (1) according the claims 1 to 9, wherein the conduit means (5) is a tube.

11. Device (1) according to claims 1 to 10, wherein the penetration means (6) is a cannula.

12. Device (1) according to claims 1 to 11, wherein the reception means (4) for the biological material comprises one or several containers.

13. Device (1) according to claims 1 to 12, wherein the reception means (4) is configured such that the biological material can be separated into at least two of its components in the reception means (4) and in particular consists of flexible, centrifugable, transparent or sterilizable material.

14. Device (1) according to claims 1 to 13, wherein the substance that is stored in the reservoir means (12) is an anti-coagulant.

15. Device (1) according to claims 1 to 14, devised as a disposable article for one-time use and/or can be sterilized.

## Revendications

1. Dispositif (1) de prélèvement de matière biologique d'un organisme, **caractérisé en ce que** ledit dispositif comprend un dispositif de déviation (3) permettant de relier au choix par lumen un dispositif de prélèvement (2)
pour le prélèvement de matière organique avec
- un dispositif de pénétration (6) pour la pénétration dans l'organisme,
- un dispositif récepteur (4) pour la réception de la matière retirée de l'organisme et
- un dispositif de stockage (12) pour l'approvisionnement d'une substance,
le dispositif de déviation (3) étant confectionné de sorte que le dispositif de prélèvement (2)
- puisse être raccordé par lumen au dispositif de stockage (12) et au dispositif récepteur (4) ou
- au dispositif de pénétration (6)

2. Dispositif selon la revendication 1, **caractérisé en ce que** la liaison par lumen est effectuée au moins partiellement par un dispositif conducteur (5).

3. Dispositif selon la revendication 1 ou 2, le dispositif de déviation (3) étant installé entre le dispositif de prélèvement (2) et le dispositif de pénétration (6).

4. Dispositif selon les revendications 1 à 3, le dispositif de déviation (3) étant installé entre le dispositif de stockage (12) et le dispositif récepteur (4).

5. Dispositif selon les revendications 1 à 4, le dispositif conducteur (5) installé entre le dispositif de stockage (12) et le dispositif de déviation (3) étant muni d'une première vanne (7) configurée de sorte que la matière biologique puisse circuler uniquement du dispositif de stockage (12) en direction du dispositif de déviation (3).

6. Dispositif selon les revendications 1 à 5, le dispositif conducteur (5) installé entre le dispositif de déviation (3) et le dispositif récepteur (4) étant muni d'une deuxième vanne (8) configurée de sorte que la matière biologique puisse circuler uniquement du dispositif de déviation (3) en direction du dispositif récepteur (4).

7. Dispositif selon les revendications 1 à 6, le dispositif conducteur (5) étant muni d'un filtre (11, 14) pour le filtrage de micro-organismes et/ou de matière biologique grumeuse.

8. Dispositif selon les revendications 1 à 7, le dispositif de prélèvement (2) étant confectionné sous forme de seringue.

9. Dispositif selon les revendications 1 à 8, le dispositif de déviation (2) étant confectionné sous forme de robinet à voies multiples, notamment sous forme de robinet à trois voies.

10. Dispositif selon les revendications 1 à 9, le dispositif (5) étant confectionné sous forme de tuyau.

11. Dispositif selon les revendications 1 à 10, le dispositif de pénétration (6) étant confectionné sous forme de canule.

12. Dispositif selon les revendications 1 à 11, le dispositif récepteur (4) accueillant la matière biologique étant composé d'un ou de plusieurs récipients.

13. Dispositif selon les revendications 1 à 12, le dispositif récepteur (4) étant confectionné de sorte que la matière biologique recueillie dans le dispositif récepteur (4) puisse être séparée en au moins deux composants, ledit dispositif étant confectionné en matièriau flexible, centrifugable, transparent ou stérilisable.

14. Dispositif selon les revendications 1 à 13, la substance approvisionnée dans le dispositif de stockage (12) étant un anticoagulant.

15. Dispositif selon les revendications 1 à 14, ledit dispositif étant conçu comme article jetable et/ou stérilisable.
